# EUROPEAN PATENT SPECIFICATION

(11) **EP 3 024 548 B1**
(45) Date of publication and mention of the grant of the patent: **21.10.2020**
(21) Application number: 14738808.6
(22) Date of filing: 09.07.2014
(51) Int. Cl.: A61N 5/10, A61M 25/01, A61B 5/06, A61B 8/08

(54) **SYSTEM FOR LOCALIZING BODY STRUCTURES**
SYSTEM ZUM AUFFINDEN VON KÖRPERSTRUKTUREN
SYSTÈME POUR LOCALISER DES STRUCTURES CORPORELLES

(30) Priority: 23.07.2013 US 201361857349 P; 14.08.2013 EP 13180388
(43) Date of publication of application: 01.06.2016
(73) Proprietor: Koninklijke Philips N.V., 5656 AG Eindhoven (NL)
(72) Inventor: TAHMASEBI MARAGHOOSH, Amir, Mohammad, 5656 AE Eindhoven (NL); VIGNON, Francois, Guy, Gerard, Marie, 5656 AE Eindhoven (NL); BHARAT, Shyam, 5656 AE Eindhoven (NL); DEHGHAN MARVAST, Ehsan, 5656 AE Eindhoven (NL); JAIN, Ameet, Kumar, 5656 AE Eindhoven (NL)
(74) Representative: Philips Intellectual Property & Standards
(86) International application number: PCT/EP2014/064688
(87) International publication number: WO 2015/010900

(56) References cited:
- WO-A1-99/05971
- WO-A1-2005/007228
- WO-A1-2011/009087
- WO-A1-2012/172458
- WO-A2-2004/101022
- US-A1- 2010 145 187
- US-A1- 2011 224 538
- US-B2- 6 587 709

## Description

### FIELD OF THE INVENTION

The invention relates to the field of systems for localizing body structures.

### BACKGROUND OF THE INVENTION

Prostate cancer is the most common organ malignancy among American men. Several treatments have been proposed for treating prostate cancer. One of them is prostate brachytherapy which refers to placement of permanent radioactive seeds or temporary radioactive sources (within hollow catheters) inside the prostate gland with insertion through the perineum. Most often, treatment planning and needle or catheter placement relies on intensive use of transrectal ultrasound imaging (TRUS).

One major drawback of brachytherapy for treatment of prostate cancer is that almost all patients develop some degree of urinary symptomatology. Urethra irritation and urinary obstruction are well documented short-term complications of the modern brachytherapy techniques. This can mainly be attributed to an unwanted incidental dose delivered to the urethra during brachytherapy procedures, which is the result of insufficient and inaccurate knowledge of the shape and pose of the urethra.

Therefore, identifying and preserving body structures, such as the prostatic urethra, is important in avoiding such post-treatment effects. To identify and localize the prosthetic urethra, different approaches have been proposed, e.g., image-based methods such as contrast Computed Tomography (CT) or magnetic resonance imaging (MRI), or the use of ultrasound visible catheters inside the urethra. However, such approaches impose additional clinical workload and increases patient financial burden, or other benefit maybe diminishing due to patient motion and image distortion caused by a patient's physiological change.

As a result, in many clinical practices, the urethra either may not be listed as an organ at risk due to the difficulty in identifying its precise localization, or may be assumed to be at a standard position with an estimated uniform margin, such as one cm.

WO 2004/101022 A2 discloses an urethral identification system, wherein a patient's urethral anatomic course is identified in real time for the precise placement of a treatment element in-to the patient's prostate, and wherein a medical toll (i.e. catheter) containing an external, in-flatable imaging bladder is introduced into a urethra of the patient until the image bladder is generally aligned with a treatment site of the prostate, an imaging probe of an imaging device is operatively positioned relative to the treatment site of the prostate and proximate portions of the urethra, the imaging device is activated so as to obtain a real time image of the treatment site of the prostate.

WO 2005/007228 A1 discloses a method of ultrasonic guidance of aiming and steering of a pacing lead with a pacing electrode into the coronary vein via the coronary sinus (CS). The procedure must include visualization of the coronary sinus, preferably by ultrasound scan-ning, localization of the pacing catheter tip, directing - steering the tip along the CS axis and thus its placement into the coronary sinus. A marking piezoelectric transducer is mounted at or near the tip of the steerable delivery catheter that can be localized in the vicinity of the coronary sinus.

US 2011/224538 A1 discloses a system and its method of use for identifying a patient's ure-thral anatomic course for the precise placement of a treatment element into the patient's prostate, wherein a echoopaque catheter is introduced into a urethra until its echogenic and radio-opaque portion is generally aligned with a treatment site of the prostate. An image probe is positioned relative to the treatment site and the urethra and is activated to obtain a real time image of the prostate, and urethra. The echo-opaque catheter may be made of an elongated flexible catheter, which is coated with, incorporate with or enclosed by materials that enhances the ultrasound and radio visualization.

WO 2011/009087 A1 discloses a heart treatment kit, system and method for radiosurgically alleviating arrhythmia. Radiosurgical treatments of tissues of the heart mitigate arrhythmias and treat other tumerous and non-tumerous disease using an implanted fiducial positioned in or near the heart using cardiac catheterization techniques. The fiducials may be implanted after diagnostic and planning images of the target tissues have been acquired. Fiducial implantation may take place the day of a scheduled radiosurgical treatment. Techniques to accommodate post-planning fiducial implantation may include registration of the implanted fiducial location with the treatment plan, and active fiducials may limit collateral imaging radiation exposure while enhancing tracking accuracy.

### SUMMARY OF THE INVENTION

It is an object of the present invention to provide a new approach for identifying and localizing body structures such as the prosthetic urethra or other critical body structures.

This object is achieved by a system as claimed in claim 1, and by a computer program product as claimed in claim 13.

Accordingly, the proposed localizing or tracking approach is based on at least one ultrasound sensor (or receiver) embodied on a medical tool to be used during an ultrasound-guided procedure. As the medical tool and ultrasound sensor enter the ultrasound field of view, the ultrasound sensor receives the ultrasound signals coming from the ultrasound probe as its beam(s) sweep the field of view. The position of the ultrasound sensor can then be determined and tracked in the frame of reference (i.e. field of view) of the ultrasound probe and the course of the critical body structure to be localized can be delineated based on the determined locations of the ultrasound sensor. This enables precise outlining of the course or shape of the critical body structure (e.g. prostatic urethra).

Moreover, the proposed localization also allows continuous real-time tracking of the critical body structure during treatment, thereby enabling adaptive treatment planning and dose delivery for better clinical outcomes (e.g. fewer urethra injury-related complications).

In general, the proposed solution as claimed enables high accuracy in localization and segmentation of critical body structures without imposing more clinical workload and cost.

According to a first aspect, the controller maybe adapted to segment and track the delineated course of the body structure and to calculate a radiation dose to be delivered to the target tissue or organ based on the tracking and segmentation. This segmenting and tracking enables real-time adjustment of radiation dose planning to minimize detrimental impact on the body structure.

According to a second aspect which can be combined with the above first aspect, a retracting unit may be provided for retracting the medical tool, the retracting unit being controlled by the controller. Thereby, the position of the embedded ultrasound sensor(s) can be controlled based on the output signal of the ultrasound sensor(s) to keep the sensor position(s) within the ultrasound field of the ultrasound probe.

According to a third aspect which can be combined with the above first or second aspect, wherein said medical tool may be a catheter. Advantageously, the flexible shape of the catheter allows insertion into and adaptation to elongated structures, so that a close match of the estimated delineation with the true shape can be achieved.

In a first specific example of the third aspect, the catheter may comprise a plurality of the ultrasound sensors mounted at a fixed distance from one another. This measure allows fixation of the catheter to or in the body structure, since the course or shape of the body structure can be interpolated based on the output signals of the equidistant ultrasound sensors.

In a second specific example of the third aspect, the catheter may comprise a guide wire to which the ultrasound sensor is attached and which can be slid in and out of the catheter. This measure also allows fixation of the catheter to or in the body structure, since the course or shape of the body structure can now be determined by sliding the ultrasound sensor through the catheter under closed-loop control based on its output signal.

According to a fourth aspect which can be combined with any one of the above first to third aspects, the controller may be adapted to determine a distance of the ultrasound sensor from the ultrasound probe based on a time of arrival of the output signal, and to determine an angular direction to the ultrasound sensor based on an amplitude of the output signal as a function of an imaging beam steering angle of the ultrasound probe. Thereby, the location of the ultrasound sensor(s) and thus the location of the body structure can readily be determined based on an analysis of the output signal(s) of the ultrasound sensor(s).

According to a fifth aspect which can be combined with any one of the above first to fourth aspects, the controller may be adapted to delineate the course of the body structure by performing an interpolation between recorded positions of the at least one ultrasound sensor tracked in the field of view. This provides a straight forward solution for delineating the course or shape of the body structure based on sensor positions determined and recorded during the sweeping operation of the ultrasound probe.

According to a sixth aspect which can be combined with any one of the above first to fifth aspects, the ultrasound probe may be mounted on an encoder to access a third dimension. Thereby, an ultrasound probe with a two-dimensional ultrasound field can be used to obtain a three-dimensional location of the ultrasound sensor.

According to a seventh aspect which can be combined with any one of the above first to sixth aspects, the ultrasound probe may be adapted to be retracted, advanced or rotated during said sweeping of said field of view. This measure enables flexible and adaptive detection of sensor output signals.

According to an eighth aspect which can be combined with any one of the above first to seventh aspects, the ultrasound probe may be a transrectal ultrasound probe, the target organ may be a prostate gland, and the body structure may be a prostatic urethra. Thus, more precise outlining of the course of the prostate urethra can be achieved.

It is noted that the controller may be implemented based on discrete hardware circuitry with discrete hardware components, an integrated chip, or an arrangement of chip modules, or based on a signal processing device or computer device or chip controlled by a software routine or program stored in a memory, written on a computer readable medium, or downloaded from a network, such as the Internet.

It shall be understood that the system of claim 1, and the computer program product of claim 13 have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described herein after.

### BRIEF DESCRIPTION OF THE DRAWINGS

In the following drawings:
Fig. 1 shows a schematic view of an anatomy of a normal prostate gland and a prostatic urethra,
Fig. 2 shows a cross section view of a portion of a patient, to which a brachytherapy procedure with real-time localization of the prostatic urethra according to a first embodiment is applied,
Fig. 3 shows a schematic architecture of a localization system with a catheter with multiple sensors according to a second embodiment,
Fig. 4 shows a schematic architecture of a localization system with a single-sensor catheter with retractor according to a third embodiment,
Fig. 5 shows a schematic architecture of a localization system with a catheter with sensors located towards a posterior side of a prostate gland, according to a fifth embodiment,
Fig. 6 shows a schematic architecture of a localization system with a catheter with ultrasound sensors in circumferential ring arrangement according to a sixth embodiment, and
Fig. 7 shows a flow diagram of a localization procedure for use in various embodiments.

### DETAILED DESCRIPTION OF EMBODIMENTS

Embodiments of the present invention are now described based on an intelligent sensing system for identifying and localizing the prostatic urethra, as an example of a critical body structure, using a US-based tracking, as an example of an ultrasound based localization or tracking technology.

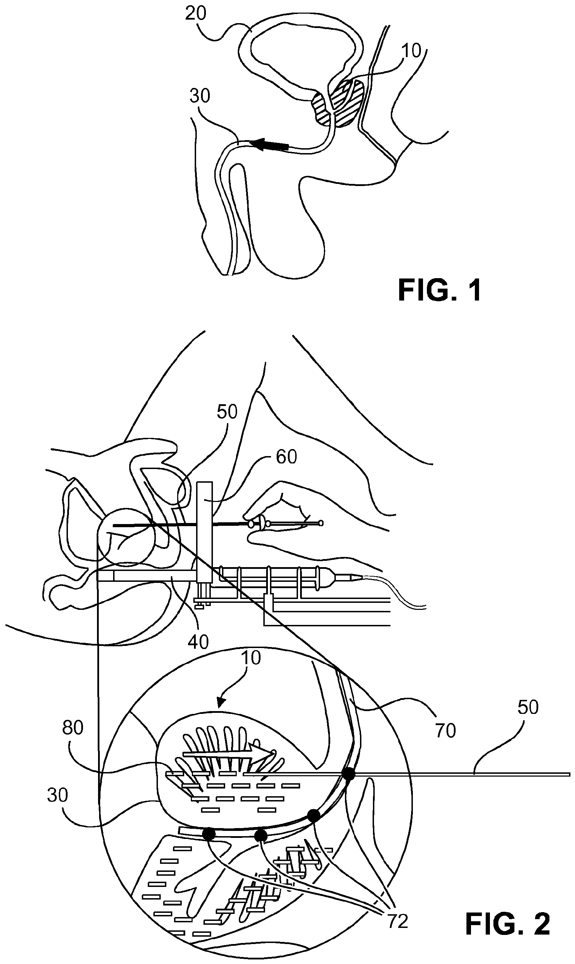

Fig. 1 shows a schematic anatomy of the prostatic urethra 30 which is approximately 3.5 cm long and passes through the prostate gland 10 to the bladder 20. Urethral complications following radiation therapy include urethritis, urethral stricture, and urethral fistula. Prostatic urethrorectal fistula has been reported to occur in 1% of patients after prostate brachytherapy for prostate cancer. By accurate knowledge of the shape and position of the prostatic urethra 30, as achieved by the present embodiments, urethral complications can be reduced substantially and detrimental effects on the prostatic urethra can be largely avoided.

Fig. 2 shows a schematic arrangement of a brachytherapy procedure with a Foley catheter 70 with integrated US sensors 72. A Foley catheter is a flexible tube that is often passed through the urethra 30 and into the bladder. The tube may have two separated channels, or lumens, running down its length. One lumen is open at both ends, and allows urine to drain out into a collection bag. The other lumen may have a valve on the outside end and may connect to a balloon at the tip. The balloon is inflated with sterile water when it lies inside the bladder, in order to stop it from slipping out. Foley catheters are commonly made from silicone rubber or natural rubber.

In the present embodiments, the use of such a Foley catheter 70 with embedded US sensors 72 enables real-time localization of the prostatic urethra 30 which is a crucial in the brachytherapy procedure to avoid unnecessary or excessive radiation dose to the prostatic urethra 30. The Foley catheter 70 is equipped with one or multiple US sensors 72 that are mounted at a fixed distance from one another in the first embodiment.

In the following, the word "catheter" is used as a generic term to denote a catheter equipped with sensors or a guide wire equipped with sensors inside a catheter.

During the brachytherapy procedure, a transrectal US (TRUS) probe 40 is used as an ultrasound signal source required for sensor-tracking. The US sensors 72 on the Foley catheter 70 receive ultrasound waves emitted by the TRUS probe 40. In particular, the ultrasound signals received from appropriate A-line(s) of the TRUS probe 40 as the beam of the TRUS probe 40 sweeps the field of view can be analyzed for determining the distance of the US sensors 72 from the TRUS probe 40 based on the time of arrival of the received ultrasound signals. The angular dimension can be determined based on the amplitude of the received ultrasound signals as a function of the imaging beam steering angle of the TRUS probe 40. The output of the US sensors 72 is fed to a control module (which may be software-based or software-controlled) for reading or receiving position and orientation data from the individual US sensors 72 using the proposed tracking technology according to the first embodiment. The trajectory of the catheter 70 is calculated using position and orientation information from all US sensors 72 in real-time.

At the beginning of the brachytherapy procedure, the tracked catheter 70 is passed through the prostatic urethra 30. The tracking technology is realized using the US sensors 72 along the catheter 70 as well as the TRUS probe 40. After implantation of the catheter 70 and release of a seed 80, a map of the delivered dose to the prostate gland 10 is (re-)calculated based on the actual position of the seed 80 or source as compared to a planned position. The seed 80 can be implanted and released by a needle 50 through a grid 60 as shown the upper part of Fig. 2.

In order to avoid excessive radiation to the prostatic urethra 30, the determined real-time trajectory of the prostatic urethra 30 is fed back to a planning control function (e.g. planning software) to recompute and update seed/source insertion paths for needles 50 yet to be inserted. As seeds 80 are dropped, the delivery dose to the prostatic urethra 30 according to real-time localization data of the prostatic urethra 30 is recomputed and if necessary, the seed dropping plan is updated accordingly.

The embodiments of the present invention vary in the number of sensors 72 on the catheter 70 and the use of a two-dimensional (2D) or three-dimensional (3D) TRUS probe 40.

In the present first embodiment, a 3D TRUS probe 40 is used. The catheter 70 may be equipped with one or more US sensors 72.

If the catheter 70 has one embedded sensor 72, the catheter 70 may be progressively inserted into or retracted from the prostatic urethra 30. As the catheter 70 is inserted into or retracted from the prostatic urethra 30, the estimated positions of the US sensor 72 are stored and represent a 3D shape and position of the catheter 70. These estimated position can be further fitted under constrains imposed by the known mechanical characteristics of the catheter 70.

If the catheter 70 has two or three embedded sensors, the estimated positions of all sensors 72 may be recorded while the catheter 70 is inserted or retracted, and multiple-shape estimates may be averaged or otherwise combined to provide the 3D shape and position of the catheter 70.

If the catheter 70 has four or more sensors 72, these sensors 72 can be distributed along the length of a portion of the catheter 70 that is located within the prostatic urethra 30, wherein proper catheter positioning can be achieved easily with catheter tracking.

The individual positions of the US sensors 72 are estimated and a polynomial or other fit to the discrete points is calculated to serve as the 3D shape and position of the catheter 70.

In general, if there are many US sensors 72, the need for recording sensor positions while retracting the catheter 70 is minimized (the more the number of sensors, the higher the accuracy of static catheter position estimation). If there are four or more sensors 72 on the catheter 70, the catheter 70 can be fixed inside the prostatic urethra 30.

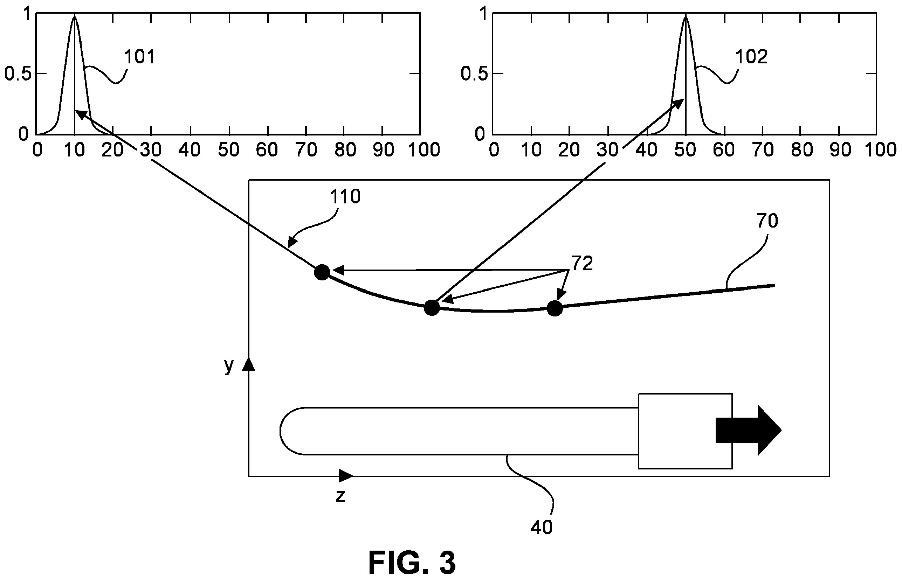

Fig. 3 shows a schematic architecture of a localization system with a catheter 70 and multiple US sensors 72 according to a second embodiment. The second embodiment can be used in connection with a 2D TRUS probe 40. A catheter 70 with four or more US sensors 72 to map the shape of the catheter 70 is used in combination with the TRUS probe 40 capable of 2D imaging only. Due to the 2D restriction, the second embodiment requires the TRUS probe 40 to be mounted on or connected to an encoder (not shown) to provide access to the third dimension. The encoder serves to electronically or mechanically control the ultrasound beam or image plane 110 of the TRUS probe 40 to achieve a 3D scanning or sweeping function over the field of view.

The US sensors 72 are distributed along the catheter length expected to be located in the prostatic urethra 30. The encoded TRUS probe 40 is then used to detect the position of the US sensors 72. To achieve this, the TRUS probe 40 can be e.g. retracted or rotated (depending on which plan is used) and the signal received by each US sensor 72 is used to detect whether the US sensor 72 is in the actual TRUS 2D image plan 110. In the example of Fig. 3, a case is shown, where the TRUS probe 40 is retracted (as indicated by the arrow).

As an alternative, the TRUS probe 40 can be in a sagittal mode and equipped with a rotation encoder. In this case, a similar approach can be used to localize the US sensors 72 by rotating the TRUS probe 40 around its axis.

When one of the US sensors 72 is within the 2D image plan of the TRUS probe 40, its location in the 2D image is combined with the probe position to reveal the 3D position of the US sensor 72.

In the upper part of Fig. 3, two time diagrams with different output signals 101, 102 of different US sensors 72 are shown, wherein the time difference on the horizontal axis can be used to determine the location of the respective US sensor 72 with regard to the 2D image plane 110 of the TRUS probe 40.

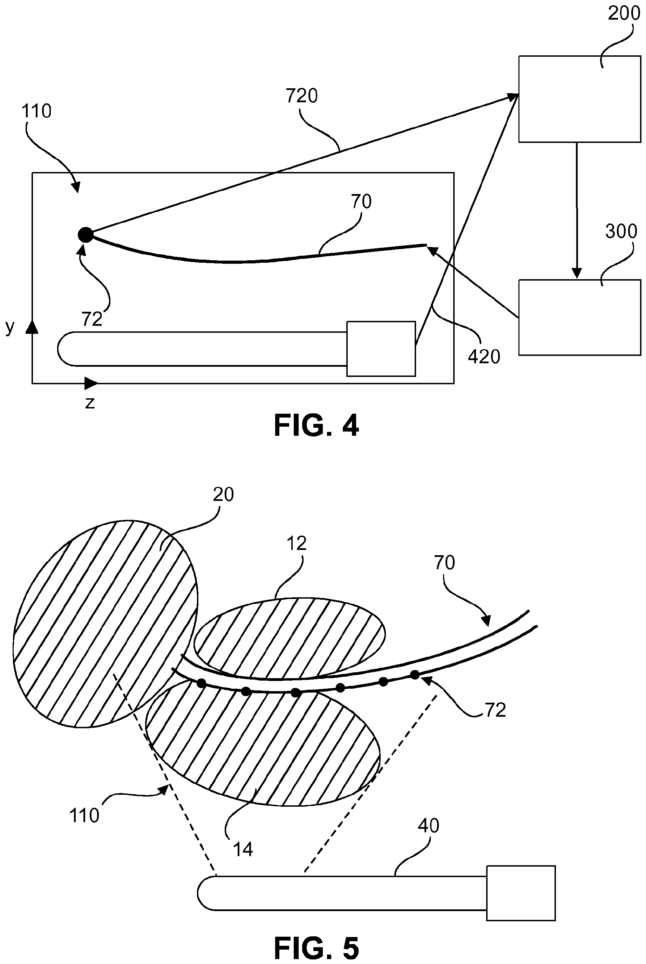

Fig. 4 shows a schematic arrangement of a localization system with a single-sensor catheter 70 in closed-loop control according to a third embodiment which can be used for 2D TRUS probes 40.

In the third embodiment, the catheter 70 has a single US sensor 72, wherein the output signal 720 of the single US sensor 72 is input to a computer or controller 200 that controls a retractor or retracting device 300 that retracts the catheter 70. The controller 200 may be controlled by a control procedure implemented as a software routine. Similarly, the encoded TRUS probe 40 may be retracted manually or automatically.

In the automatic system with closed-loop control, the retractor 300 can be controlled by the controller 200 based on the sensor output signal 720 and a probe position signal 420 received from the TRUS probe 40, in a way that the US sensor 72 is always kept in the 2D image plan of the moving TRUS probe 40. Then, the 2D position of the US sensor 72 in the TRUS image can be combined with the position of the tracked TRUS probe 40 to reveal the 3D position of the US sensor 72.

According to a fourth embodiment, the controller 200 may control a robot (not shown) that advances or retracts the TRUS probe 40. Additionally, the sensor-equipped catheter 70 is retracted or advanced by the retracting device 300, as shown in Fig. 4. The probe-holding robot is controlled in a way that the US sensor 72 is always kept in the 2D image of the moving TRUS probe 40. Again, the 2D position of the US sensor 72 in the TRUS image can be combined with the position of the TRUS probe to reveal the 3D position of the US sensor 72.

Fig. 5 shows a schematic architecture of a localization system according to a fifth embodiment which is adapted to ensure signal reception even in the presence of air pockets in the prostatic urethra 30.

To account for possible air pockets in the catheter 70, that could impede or hinder ultrasound propagation at the surface of the catheter 70 proximal to the posterior side or part 14 of the prostate gland, all US sensors 72 may be attached to the catheter 70 in the same orientation along its length. A visible marking on the outer end of the catheter 70 may indicate where along its circumference the multiple US sensors 72 are located. During insertion of the catheter 70, it can be ensured that this marking always points towards the posterior side of the patient, i.e., proximal to the posterior part 14 of the prostate gland 10, as shown in Fig. 5.

Since the catheter 70 usually does not twist or torque, this external marking is a suitable surrogate for the positions of the US sensors 72. Fig. 5 also shows the 2D image 110 of the TRUS probe which may be swept electronically without any retraction or advancement of the TRUS probe 40.

Above the catheter 70, the interior part or side 12 of the prostate 30 is shown.

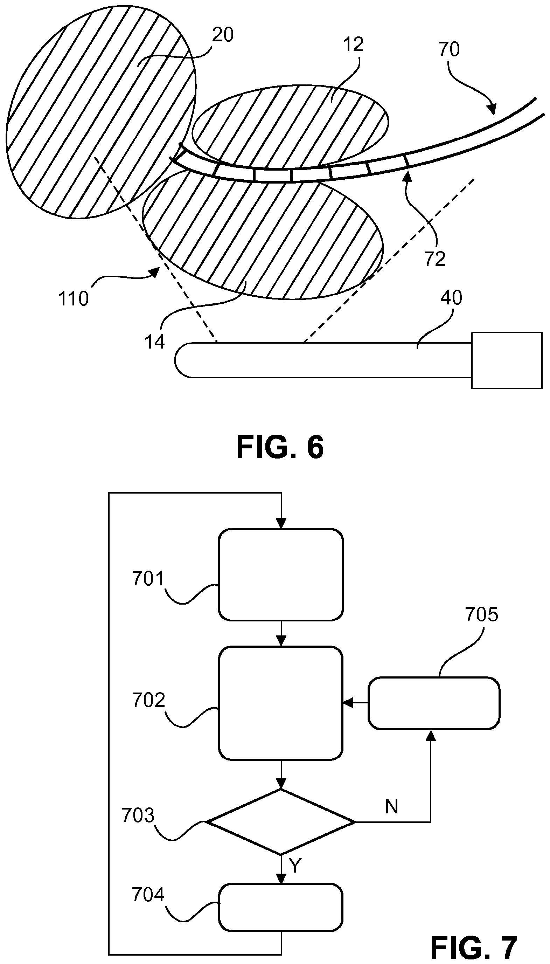

Fig. 6 shows an arrangement of a localization system according to the sixth embodiment which is similar to the fifth embodiment of Fig. 5 with the exception that the US sensors 72 are configured as circumferential rings to ensure signal reception even in the presence of air pockets in the urethra 30.

The catheter 70 of the sixth embodiment provides the advantage that a twist or torque of the catheter 70 is not harmful, since a quantifiable output signal of the US sensors 72 can be obtained regardless of the orientation of the catheter 70.

Alternatively, a small sensor-equipped guide wire may be constructed in a manner that it can be slit in an out of the hollow channel of the catheter 70. The guide wire may be small, or hollow, so as not to obstruct the flow of urine through the catheter 70. Moreover, if the catheter 70 is equipped with the guide wire, the circumferential ring shape can be used for a sensor attachment to the guide wire. A closed-loop control can then be achieved similar to the third embodiment of Fig. 4, wherein the retracting device 300 may now control the movement of the guide wire.

The 3D catheter shape and position and an estimated diameter of the urethra 30 can be used to segment the prostatic urethra 30. To achieve this, the outer diameter of the catheter 70 can be utilized as an estimate of the diameter of the urethra 30, assuming a snug fit of the catheter 70 within the prostatic urethra 30. Therefore, the diameter of the prostatic urethra 30 can be added to the estimated positions of the US sensors 72 (which represent the proximal/posterior edge of the prostatic urethra 30) to obtain the distal/anterior edge of the prostatic urethra 30. Then, by sticking multiple circles having a diameter which corresponds to the diameter of the prostatic urethra 30 at each measurement point and interpolating these circles, a 3D segmentation of the urethra 30 can be obtained.

Additionally, the estimated 3D catheter shape and position can be used to estimate the location and position of the prostate gland 10. At the beginning of the brachytherapy procedure, the prostate gland 10 can be localized in the field of view or frame of reference of the TRUS probe 40. However, during the brachytherapy procedure, the prostate gland 10 may move. To adapt the brachytherapy procedure to such moves, the estimated position and shape of the urethra 30 can be used to automatically update the location of the prostate gland 10 in the frame of reference of the TRUS probe 40 and use it for adaptive treatment planning and delivery.

The planning software or planning control function can be adapted to display an overlay of the tracked position of the prostatic urethra 30 or the prostate gland 10 on the user interface (e.g. screen) so that the track position is easily detectable by the user. The planning process prior to implantation ensures that source placement can be optimized to maximize target coverage while minimizing dose to organs at risk taking into account internal dose inhomogeneities. It also allows for a precise seed ordering. Planning according to defined parameters ensures reproducibility between different centers and operators.

Fig. 7 shows a flow diagram of a real-time localization and dose adaptation procedure which can be implemented in at least some of the above embodiments.

In step 701, real-time urethra segmentation and tracking as described above is performed based on tracking data which may be stored by the controller 200 of Fig. 4. The actual estimated 3D position of the prostatic urethra 30 is then used in step 702 to compute a radiation dose just before seed deposition.

In step 703, it is then checked whether the computed radiation dose of step 702 is adequate under consideration of the estimated real-time position of the prosthetic urethra. If so, a seed with the computed radiation dose is dropped and the procedure jumps back to step 701. Otherwise, if it is determined in step 703 that the computed radiation dose is critical for the prosthetic urethra 30, the procedure jumps to step 705 and the delivery plan is adjusted to reduce dose input to the prosthetic urethra 30.

The procedure of Fig. 7 thus allows adaptive re-planning of prostate brachytherapy based on real-time localization and tracking of the prostatic urethra.

To summarize, a localization system and method have been described, in which a Foley catheter or other medical tool which is equipped with US sensor(s) is inserted into the prostatic urethra. Based on analysis of the US signal received by these US sensors as the US beams from a TRUS probe or other ultrasound probe sweep the field of view, it is possible to precisely detect and track these US sensors in the same frame of reference as the TRUS images, thereby precisely delineating the Foley catheter and the course of the prostatic urethra. During the procedure, before each seed is dropped, the delivered dose to the prostatic urethra can be computed based on real-time tracking and segmentation of prostatic urethra and dose radiation based on previously dropped seeds and if necessary, the procedure can be re-planned automatically.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. A single processor or other unit may fulfill the functions of several items recited in the claims. The mere fact that certain measures are recited in mutually different dependent claims does not indicate that a combination of these measures cannot be used to advantage.

A single unit or device may fulfill the functions of several items recited in the claims. The mere fact that certain measures are recited in mutually different dependent claims does not indicate that a combination of these measures cannot be used to advantage.

The described operations of functional components of the system according to various embodiments, e.g. as described in connection with Fig. 7, can be implemented as program code means of a computer program and/or as dedicated hardware. The code means are arranged for producing at least some of the described functional steps when run on a computing device. The computer program may be stored and/or distributed on a suitable medium, such as an optical storage medium or a solid-state medium, supplied together with or as part of other hardware, but also be distributed in other forms such as via the Internet or other wired or wireless telecommunication systems.

## Claims

1. A system for localizing a body structure (30) in dosimetry of a target tissue or organ (10), said system comprising:
an ultrasound probe (40) for sweeping a field of view of said target tissue or organ (10);
a medical tool (70) comprising at least one embedded ultrasound sensor (72) for insertion in said body structure (30); and
a controller (200) for determining locations of the at least one ultrasound sensor (72) based on output signals of said at least one ultrasound sensor (72) during said sweeping and for delineating a course of said body structure (30) based on the determined locations of said at least one ultrasound sensor (72).

2. The system of claim 1, wherein said controller (200) is adapted to track and segment said delineated course of said body structure (30) and to calculate a radiation dose to be delivered to said target tissue or organ (10) based on the tracking and segmentation.

3. The system of claim 1, further comprising a retracting unit (300) for retracting said medical tool (70), said retracting unit (300) being controlled by said controller (200).

4. The system of claim 1, wherein said medical tool is a catheter (70).

5. The system of claim 4, wherein said catheter (70) comprises a plurality of said ultrasound sensors (72) mounted at a fixed distance from one another.

6. The system of claim 4, wherein said catheter (70) comprises a guide wire to which said ultrasound sensor (72) is attached and which can be slid in and out of said catheter (70).

7. The system of claim 4, wherein said catheter (70) is adapted to be fixed in said body structure (30).

8. The system of claim 1, wherein said controller (200) is adapted to determine a distance of said ultrasound sensor (72) from said ultrasound probe (40) based on a time of arrival of said output signal, and to determine an angular direction to said ultrasound sensor (72) based on an amplitude of said output signal as a function of an imaging beam steering angle of said ultrasound probe (40).

9. The system of claim 1, wherein said controller (200) is adapted to delineate said course of said body structure (30) by performing an interpolation between recorded positions of said at least one ultrasound sensor (72) tracked in said field of view.

10. The system of claim 1, wherein said ultrasound probe (40) is mounted on an encoder to access a third dimension.

11. The system of claim 1, wherein said ultrasound probe (40) is adapted to be retracted, advanced or rotated during said sweeping of said field of view.

12. The system of claim 1, wherein said ultrasound probe is a transrectal ultrasound probe (40), wherein said target organ is a prostate gland (10), and wherein said body structure is a prostatic urethra (30).

13. A computer program product comprising code means for causing the system for localizing a body structure (30) as defined in claim 1 to carry out steps of a method of localizing a body structure (30) in dosimetry of a target tissue or organ (10), when the computer program product is run on a computing device controlling the system for localizing a body structure (30), said method comprising:
- sweeping a field of view of said target tissue or organ (10) by an ultrasound probe (40);
- determining locations of at least one ultrasound sensor (72) embedded in a medical tool (70) inserted in said body structure, based on output signals of said at least one ultrasound sensor (72) during said sweeping; and
- delineating a course of said critical body structure (30) based on the determined location of said at least one ultrasound sensor (72).

## Patentansprüche

1. Ein System zum Verorten der Körperstruktur (30) eines Zielgewebes oder -organs (10) in Dosimetrie, wobei das System Folgendes umfasst:
eine Ultraschallsonde (40) zum Durchlaufen eines Bildfelds des Zielgewebes oder -organs (10);
ein medizinisches Instrument (70), das mindestens einen integrierten Ultraschallsensor (72) zum Einführen in die besagte Körperstruktur (30) umfasst; und
eine Steuerung (200) zum Ermitteln der Positionen des mindestens einen Ultraschallsensors (72) beruhend auf den Ausgangssignalen des mindestens einen Ultraschallsensors (72) beim Durchlaufen sowie Darstellen des Verlaufs der Körperstruktur (30) beruhend auf den ermittelten Positionen es mindestens einen Ultraschallsensors (72).

2. Das System gemäß Anspruch 1, wobei die Steuerung (200) den dargestellten Verlauf der Körperstruktur (30) nachverfolgt und segmentiert und beruhend auf der Nachverfolgung und Segmentierung die Strahlendosis für das Zielgewebe oder -organ (10) berechnet.

3. Das System gemäß Anspruch 1, das zudem eine Rückholeinheit (300) zum Einfahren des medizinischen Instruments (70) umfasst, wobei die Rückholeinheit (300) von der Steuerung (200) gesteuert wird.

4. Das System gemäß Anspruch 1, wobei es sich bei dem medizinischen Instrument um einen Katheter (70) handelt.

5. Das System gemäß Anspruch 4, wobei der Katheter (70) mehrere der Ultraschallsensoren (72) umfasst, die in einem festen Abstand zueinander angebracht sind.

6. Das System gemäß Anspruch 4, wobei der Katheter (70) einen Führungsdraht umfasst, an dem die Ultraschallsensoren (72) angebracht sind, und der in den und aus dem Katheter (70) geführt werden kann.

7. Das System gemäß Anspruch 4, wobei der Katheter (70) an der besagten Körperstruktur (30) befestigt werden kann.

8. Das System gemäß Anspruch 1, wobei die Steuerung (200) den Abstand des Ultraschallsensors (72) von der Ultraschallsonde (40) beruhend auf dem Erhalt des Ausgangssignals ebenso ermittelt, wie die Winkelrichtung des Ultraschallsensors (72). Zweiteres erfolgt beruhend auf der Amplitude des Ausgangssignals als Funktion des Lenkwinkels für den Bildgebungsstrahl der Ultraschallsonde (40).

9. Das System gemäß Anspruch 1, wobei die Steuerung (200) den Verlauf der Körperstruktur (30) darstellt, indem sie eine Interpolation der aufgezeichneten Positionen des mindestens einen, im Bildfeld nachverfolgten Ultraschallsensors (72) durchführt.

10. Das System gemäß Anspruch 1, wobei die Ultraschallsonde (40) an einem Messgeber angebracht ist, um eine dritte Dimension erfassen zu können.

11. Das System gemäß Anspruch 1, wobei die Ultraschallsonde (40) beim Durchlaufen des Bildfelds eingefahren, ausgefahren oder gedreht werden kann.

12. Das System gemäß Anspruch 1, wobei es sich bei der Ultraschallsonde um eine transrektale Ultraschallsonde (40) handelt, und wobei es sich beim Zielorgan um eine Prostata (10) handelt, und wobei es sich bei der Körperstruktur um eine prostatische Harnröhre (30) handelt.

13. Ein Computerprogrammprodukt mit Code, der das System zum Verorten einer Körperstruktur (30) gemäß Anspruch 1 dazu veranlasst, die Schritte einer Methode zum Verorten einer Körperstruktur (30) in Dosimetrie eines Zielgewebes oder Organs (10) auszuführen, wenn das Computerprogrammprodukt auf einem Computer ausgeführt wird, der das System zum Verorten einer Körperstruktur (30) steuert, wobei die Methode folgende Schritte umfasst:
- Durchlaufen eines Bildfelds des Zielgewebes oder Organs (10) mit einer Ultraschallsonde (40);
- Ermitteln der Positionen mindestens eines in ein medizinisches Instrument (70) integrierten und die Körperstruktur eingeführten Ultraschallsensors (72) beruhend auf den Ausgangssignalen des mindestens einen Ultraschallsensors (72) beim Durchlaufen; und
- Darstellen des Verlaufs der besagten Körperstruktur (30) beruhend auf der ermittelten Position des mindestens einen Ultraschallsensors (72).

## Revendications

1. Système de localisation d'une structure corporelle (30) en dosimétrie d'un tissu ou d'un organe cible (10), ledit système comprenant :
une sonde ultrasonore (40) pour balayer un champ de vision dudit tissu ou dudit organe cible (10) ;
un outil médical (70) comprenant au moins un capteur à ultrasons (72) intégré destiné à l'insertion dans ladite structure corporelle (30) ; et
un dispositif de commande (200) pour déterminer des emplacements de l'au moins un capteur à ultrasons (72) en fonction des signaux de sortie dudit au moins un capteur à ultrasons (72) lors dudit balayage et
pour tracer une trajectoire de ladite structure corporelle (30) en fonction des emplacements déterminés dudit au moins un capteur à ultrasons (72).

2. Système selon la revendication 1, dans lequel ledit dispositif de commande (200) est conçu pour suivre et pour segmenter ladite trajectoire délimitée de ladite structure corporelle (30) et pour calculer une dose de rayonnement à délivrer audit tissu ou audit organe cible (10) en fonction du suivi et de la segmentation.

3. Système selon la revendication 1, comprenant en outre une unité de rétraction (300) pour rétracter ledit outil médical (70), ladite unité de rétraction (300) étant commandée par ledit dispositif de commande (200).

4. Système selon la revendication 1, dans lequel ledit outil médical est un cathéter (70).

5. Système selon la revendication 4, dans lequel ledit cathéter (70) comprend une pluralité desdits capteurs à ultrasons (72) montés à une distance fixe les uns des autres.

6. Système selon la revendication 4, dans lequel ledit cathéter (70) comprend un fil de guidage auquel ledit capteur à ultrasons (72) est fixé et lequel peut être glissé dans et hors dudit cathéter (70).

7. Système selon la revendication 4, dans lequel ledit cathéter (70) est conçu pour être fixé dans ladite structure corporelle (30).

8. Système selon la revendication 1, dans lequel ledit dispositif de commande (200) est conçu pour déterminer une distance dudit capteur à ultrasons (72) de ladite sonde ultrasonore (40) en fonction d'une heure d'arrivée dudit signal de sortie, et pour déterminer une direction angulaire pour ledit capteur à ultrasons (72) en fonction d'une amplitude dudit signal de sortie en fonction d'un angle de direction du faisceau d'imagerie de ladite sonde ultrasonore (40).

9. Système selon la revendication 1, dans lequel ledit dispositif de commande (200) est conçu pour délimiter ledit parcours de ladite structure corporelle (30) par réalisation d'une interpolation entre les positions enregistrées dudit au moins un capteur à ultrasons (72) suivis dans ledit champ de vision.

10. Système selon la revendication 1, dans lequel ladite sonde ultrasonore (40) est montée sur un codeur pour accéder à une troisième dimension.

11. Système selon la revendication 1, dans lequel ladite sonde ultrasonore (40) est conçue pour être rétractée, avancée ou tournée lors dudit balayage dudit champ de vision.

12. Système selon la revendication 1, dans lequel ladite sonde ultrasonore est une sonde ultrasonore transrectale (40), dans lequel ledit organe cible est une glande prostatique (10), et dans lequel ladite structure corporelle est un urètre prostatique (30).

13. Produit de programme informatique comprenant un moyen de code permettant d'amener le système de localisation d'une structure corporelle (30) tel que défini dans la revendication 1 à mettre en œuvre les étapes d'un procédé de localisation d'une structure corporelle (30) en dosimétrie d'un tissu ou d'un organe cible (10) lorsque ledit produit de programme informatique est exécuté sur un dispositif informatique commandant ledit système de localisation d'une structure corporelle (30), ledit procédé comprenant :
- le balayage d'un champ de vision dudit tissu ou dudit organe cible (10) par une sonde ultrasonore (40) ;
- la détermination des emplacements d'au moins un capteur à ultrasons (72) intégré dans un outil médical (70) inséré dans ladite structure corporelle, en fonction des signaux de sortie dudit au moins un capteur à ultrasons (72) lors dudit balayage ; et
- la délimitation d'un parcours de ladite structure corporelle (30) critique en fonction de l'emplacement déterminé dudit au moins un capteur à ultrasons (72).
